# EUROPEAN PATENT SPECIFICATION

(11) **EP 1 647 837 B1**
(45) Date of publication and mention of the grant of the patent: **13.08.2008**
(21) Application number: 05022333.8
(22) Date of filing: 13.10.2005
(51) Int. Cl.: G01S 15/89, A61B 8/08

(54) **Ultrasound diagnostic system for providing elastic image with additional information**
Diagnostisches Ultraschallsystem zur Widergabe eines elastischen Bildes und zusätzlicher Information
Système de diagnostique à ultrasons pour fournir une image élastique avec des informations supplémentaires

(30) Priority: 15.10.2004 KR 2004082803; 12.08.2005 KR 2005074160
(43) Date of publication of application: 19.04.2006
(73) Proprietor: MEDISON CO., LTD., Kangwon-do 250-870 (KR)
(72) Inventor: Kim, Cheol An, Guseong-eu Yongin-si Gyeonggi-do 449-939 (KR); Kim, Jong Sik, Songpa-gu Seoul 138-783 (KR); Song, Young Seuk, Gangnam-gu, Seoul 135-847 (KR); Cho, Gae Young, Dobong-gu Seoul 132-850 (KR); Yoon, Ra Young, Gwanak-gu Seoul 151-810 (KR)
(74) Representative: Lorenz, Werner

(56) References cited:
- US-A- 5 293 870
- US-A- 5 993 389
- US-A1- 2004 059 224
- US-A1- 2004 143 189
- MATRE K ET AL: "In vitro evaluation of ultrasound Doppler strain rate imaging: modification for measurement in a slowly moving tissue phantom" ULTRASOUND IN MEDICINE AND BIOLOGY, NEW YORK, NY, US, vol. 29, no. 12, December 2003 (2003-12), pages 1725-1734, XP004482540 ISSN: 0301-5629

## Description

The present invention generally relates to an ultrasound diagnostic system, and more particularly to an ultrasound diagnostic system adapted to provide an elastic image together with additional information.

Currently, an ultrasound diagnostic system is widely used to inspect a target object for diagnostic purposes. In order to provide an image of a target object, the ultrasound diagnostic system radiates an ultrasound signal to the target object, receives the ultrasound signal reflected from a discontinuous surface of the target object, converts the received ultrasound signal into electrical signals and performs signal processing upon the electrical signals.

Typically, an elastic imaging technique is used to visualize a characteristic of a tissue in ultrasound medical imaging. Specifically, the elastic imaging technique is utilized because it is widely recognized in the art that the elasticity of the tissue is relevant to a pathological phenomenon. For example, a cancer or a tumor tissue is harder than a general soft tissue. Thus, the cancer or the tumor tissue is less likely to be strained than the general soft tissue against a same amount of external force.

Generally, it is necessary to obtain data before and after the constriction to measure the elasticity of the tissue. When obtaining the data from a real time ultrasound device, the strained amount of the tissue may vary depending on the constriction force applied by the user, wherein the quality of the elastic image depends on the constriction force and the frame rate of the device. More specifically, in case of a small constriction force, a relative strained amount between media, each having a different elasticity, is not displayed clearly. In case of a large strained amount, however, the quality of the elastic image becomes degraded because a decorrelation in the tissue becomes increased by the constriction. Therefore, an appropriate level of constriction is required to obtain data from a real time ultrasound device. Since the constriction strength varies with the user or the measurement, the user should be provided with information as to which level of constriction should be exerted or has been exerted. The level of constriction can be represented by the constriction speed, which can be calculated from the current frame rate and the currently computed displacement of the tissue.

In a conventional elastic imaging technique, a relative strained amount of the tissue is represented through using a pseudo color of two-dimensional (2D) image. Accordingly, the conventional elasticity imaging technique is highly helpful to diagnose a diseased portion, which cannot be detected in a B-mode image where a reflection coefficient is represented by using an impedance difference between the tissues.

However, although the conventional elastic imaging technique has the advantage of being able to visually display the hardness of the tissue, it cannot provide any precise information on the strain ratio. Therefore, in order to obtain numerical information on the strain ratio, a separate method for displaying a numerical value instead of the image is required.

In the conventional elastic imaging technique, constrictions and expansions are repeatedly performed to achieve a real time elastic image. Due to such constrictions and expansions, a flash noise is created in case the elastic image is displayed with a color Doppler image or a power Doppler image. Therefore, a method for removing the flash noise is necessary.

Regarding the relevant state of the art, reference is made to US 2004/059224A1, US 2004/143189A1 and US-A-5 993 389, the latter disclosing a device according to the preamble of claim 1.

It is, therefore, an object of the present invention to provide an ultrasound diagnostic system for displaying an elastic image along with additional information including the constriction speed and the strain ratio by the constriction, which are used to obtain the elastic image. Further, it is another object of the present invention to provide an ultrasound diagnostic system for generating the additional information while producing the elastic image together with the ultrasonic color Doppler image or the power Doppler image.

In accordance with the present invention, the ultrasound diagnostic system includes: a probe for receiving first echo signals from a target object, the probe being configured to transfer a pressure applied by an user to the target object and further receive second echo signals from the target object; an elastic image processor for producing an elastic image and additional information related thereto based on the first and second echo signals; a Doppler image processor for producing a Doppler image and a Doppler map based on the echo signals; a synthesis image processor for producing a synthesized image by synthesizing the elastic image and the Doppler image, the synthesis image processor being configured to receive the additional information and information on the Doppler map; and a display unit for displaying the synthesized image, the additional information and the Doppler map, which are inputted from the synthesis image processor.

The above and other objects and features in accordance with the present invention will become apparent from the following descriptions of preferred embodiments given in conjunction with the accompanying drawings, in which:
Fig. 1 is a block diagram showing a configuration of an ultrasound diagnostic system.
Fig. 2A shows an elastic image map representing a relationship between a strain and a constriction speed.
Fig. 2B is an exemplary diagram depicting an implementation of the elastic image of Fig. 2A.
Fig. 3A is an exemplary diagram showing a state in which the user selects a line SL on the elastic image.
Fig. 3B is a graph showing a strain ratio according to a depth of the selected line of Fig. 3A.
Fig. 4 is an exemplary diagram presenting a numerically computed strain ratio of a selection region on the elastic image.
Fig. 5 is a block diagram showing a configuration of an ultrasound diagnostic system constructed in accordance with the present invention.
Fig. 6 is an exemplary diagram showing a new frame formed by an interpolation in comparison with a frame having a noise over a threshold value.
Fig. 7 is an exemplary diagram showing a synthesized image of the elastic image and the color Doppler image.
Fig. 8 is an exemplary diagram depicting an image resulting from synthesizing the elastic image and the power Doppler image.
Fig. 9 is an exemplary diagram presenting a strain ratio of the elastic image, the constriction speed and the blood flow information of the Doppler as a single color map.
Fig. 10 is an exemplary diagram separately representing the Doppler map and the elastic image map showing the strain ratio of the elastic image and the constriction speed.

Hereinafter, the embodiments of the present invention will be described by referring to the attached drawings.

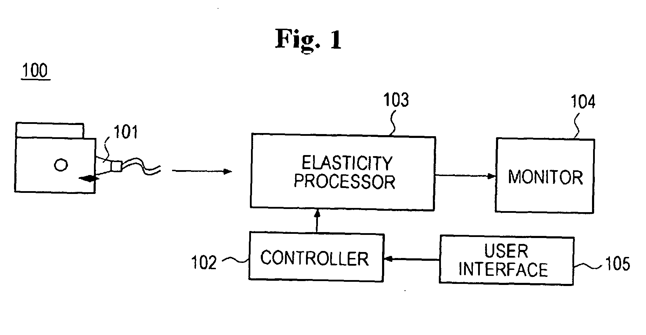

Referring to Fig. 1, an ultrasound diagnostic system 100, includes a probe 101, a controller 102, an elastic image processor 103, a monitor 104 and a user interface unit 105.

The probe 101 transmits an ultrasound signal to a target object and receives an ultrasonic signal (echo signal) reflected from the target object. The probe is also configured to transfer a pressure applied by a user to the target object.

The controller 102 controls an overall operation of the ultrasound diagnostic system 100. Particularly, the controller 102 provides a previously set frame rate to the elastic image processor 103.

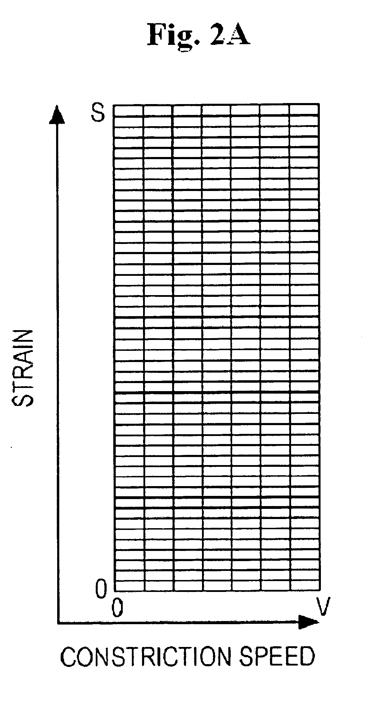

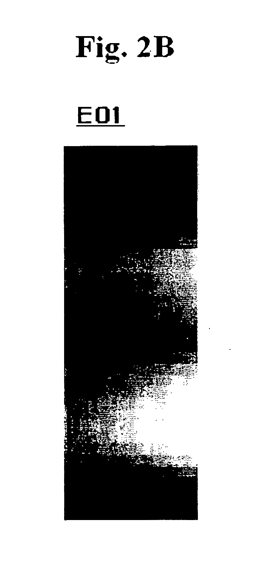

The elastic image processor 103 calculates a strain ratio of a medium based on the echo signal inputted from the probe 101. That is, the elastic image processor 103 calculates the strain ratio of the medium based on a change in the echo signals before and after applying the pressure. Additionally, the elastic image processor 103 calculates a constriction speed based on the calculated strain ratio and the frame rate information provided by the controller 102 and makes an elastic image map E01 (i.e., a pseudo color map showing information on the constriction speed and the strain ratio). The constriction speed (mm/sec) can be calculated by multiplying the frame rate (frames/sec) and a displacement (mm/frame) computed from a current frame. Fig. 2A shows an elastic image map representing a relationship between the strain and the constriction speed in accordance with one embodiment of the present invention. Fig. 2B is an exemplary diagram depicting an implemented aspect of Fig. 2A. As shown in Figs. 2A and 2B, an entire span of the constriction speed may be divided into predetermined number of steps. The information on the strain ratio can be represented through using a 256 or 512 number of steps.

The monitor 104 is provided with the above-mentioned elastic image map showing the information on the constriction speed and the strain ratio.

The strain ratio of the region selected by the user may be provided in the form of numerical values. That is, if the user selects any region from the elastic image through the user interface unit 105, the numerical information on the strain ratio of the selected region is provided.

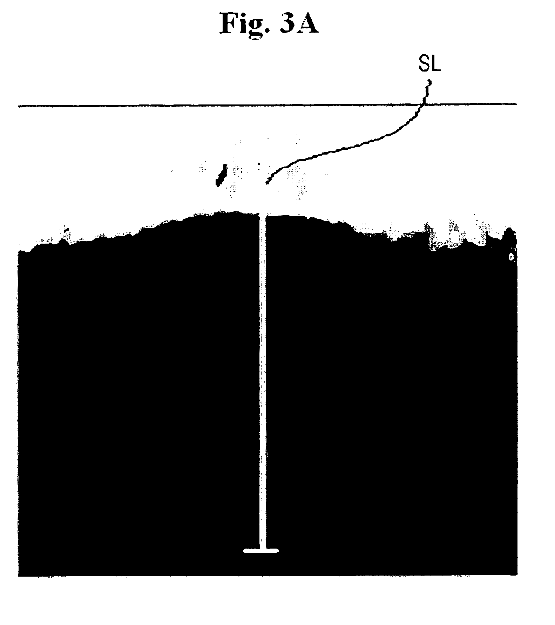

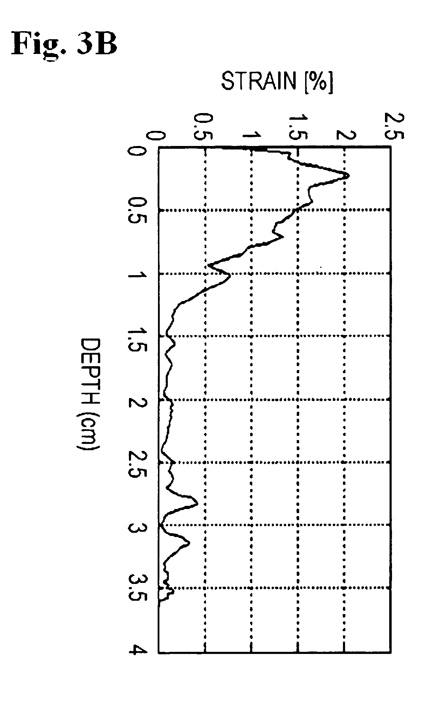

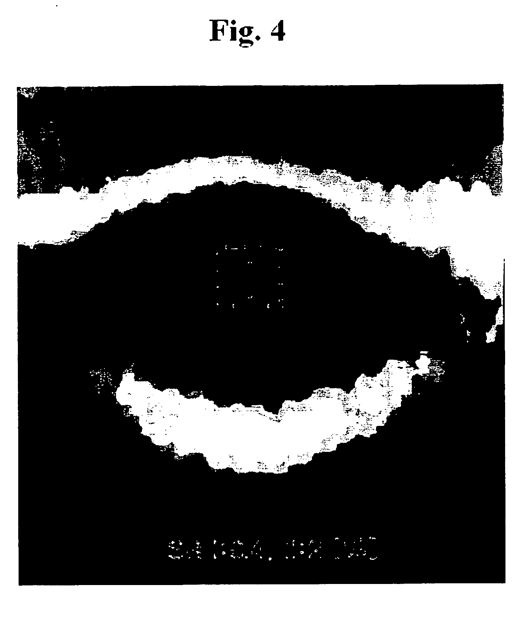

Fig. 3A is an exemplary diagram showing a state in which the user selects a line SL on the elastic image and Fig. 3B is a graph showing the strain ratio according to a depth of the selected line of Fig. 3A. As shown in Fig. 4, in case the user selects two random regions, the strain ratios of the selected regions (I and II) are calculated and accordingly the numerical information is provided to the user. The numerical information may include a variance, an average and a standard deviation of the strain ratio, wherein the strain ratio versus a position in the selected region can be provided in the form of a graph.

The Doppler image displays an amount of frequency shift together with a diffusion intensity of the signal so that a dynamic function of a living body can be observed. Particularly, the color Doppler and the power Doppler images are obtained by demodulating the received signal and performing digital processing thereon so as to depict a blood flow in a heart or a large blood vessel as a real time 2-D image.

In the color Doppler image a cross section of tissue is presented by a black and white B-mode image and the blood flow is displayed with colors. The blood flows running into and against a direction of the radiated ultrasonic beam are displayed with warm color (red series) and cold color (blue series), respectively. The power Doppler image can represent information on the blood flow by displaying the power of the blood flow with a different brightness of color.

In accordance with another embodiment, the ultrasound diagnostic system is provided, which presents an image with the Doppler image and the elastic image synthesized, together with the constriction speed of the target object set at the time of obtaining the elastic image. The ultrasound diagnostic system according to this embodiment facilitates the exact diagnosis of a diseased portion.

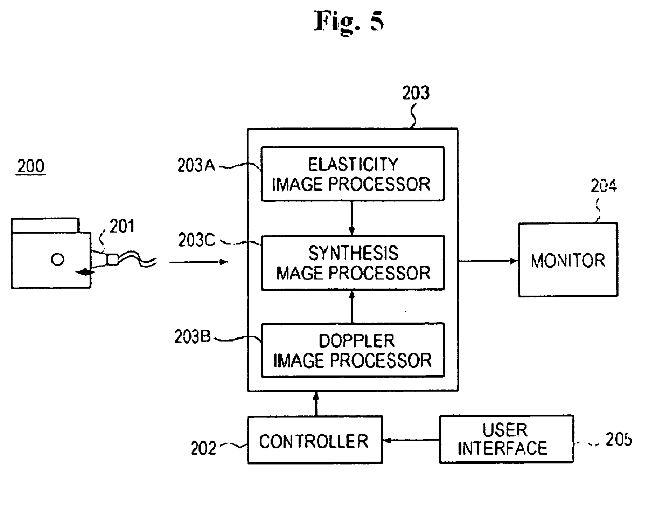

As shown in Fig. 5, an ultrasound diagnostic system 200, which is constructed in accordance with the present invention, includes a probe 201, a controller 202, an image processor 203, a monitor 204 and a user interface unit 205. The image processor 203 includes an elastic image processor 203A, a Doppler image processor 203B and a synthesis image processor 203C.

Since the functions of the probe 201, the controller 202, the elastic image processor 203A, the monitor 204 and the user interface unit 205 are identical to those of the probe 101, the controller 102, the elastic image processor 103 and the monitor 104 and the user interface 105, respectively, the detailed descriptions of the above-mentioned elements are omitted herein.

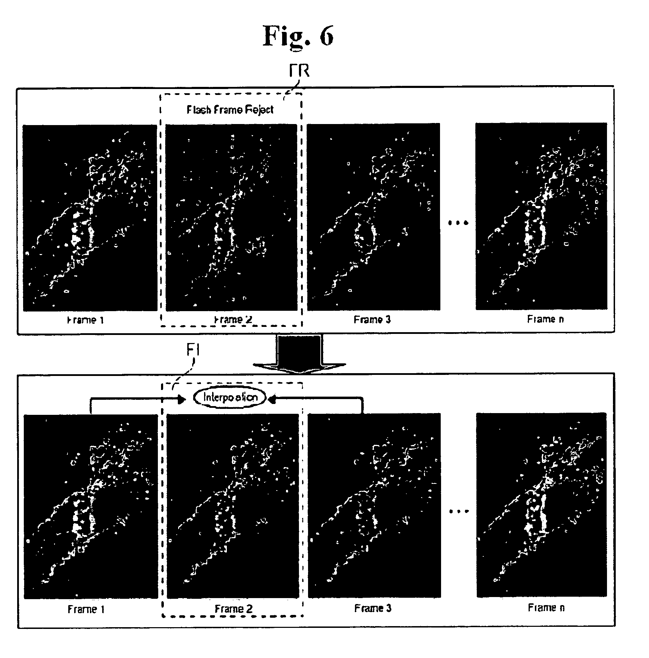

The Doppler image processor 203B corresponds to the color Doppler image processor or the power Doppler image processor. The Doppler image processor 203B produces the color Doppler image/the power Doppler image (hereinafter referred to as the Doppler image) and the Doppler image map based on the echo signal inputted from the probe 201. The Doppler image map shows the speed or the power of the blood flow by a color map. Due to the repeated constriction and restoration of the target object for obtaining the elastic image, the flash noise may occur to the Doppler image. For each frame of the Doppler image obtained, the Doppler image processor 203B checks the quantity of a flash noise occurrence, removes the frame having the flash noise over a threshold value and replaces the removed frame with a new frame formed by an interpolation by using frames before and after the removed frame. Fig. 6 shows the new frame FI formed by the interpolation in comparison with the frame FR having a noise over the threshold value.

The synthesis image processor 203C produces the synthesized image by synthesizing the elastic image and the Doppler image delivered from the elastic image processor 203A and the Doppler image processor 203B, respectively. The synthesis image processor 203C provides the monitor 204 with the Doppler image map received from the Doppler image processor 203B and at least one of the strain ratio, the constriction speed and the pseudo color map from the elastic image processor 203A, together with the synthesized image.

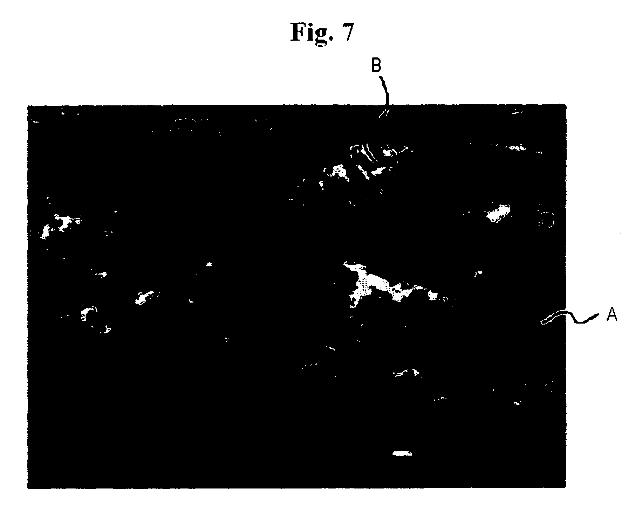

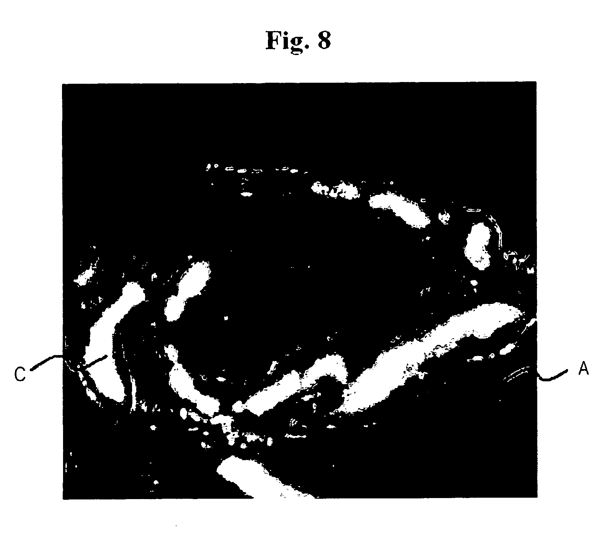

Fig. 7 is an exemplary diagram showing the image resulting from synthesizing the elastic image A and the color Doppler image B. Fig. 8 is an exemplary diagram depicting the image A with the elastic image and the power Doppler image C synthesized.

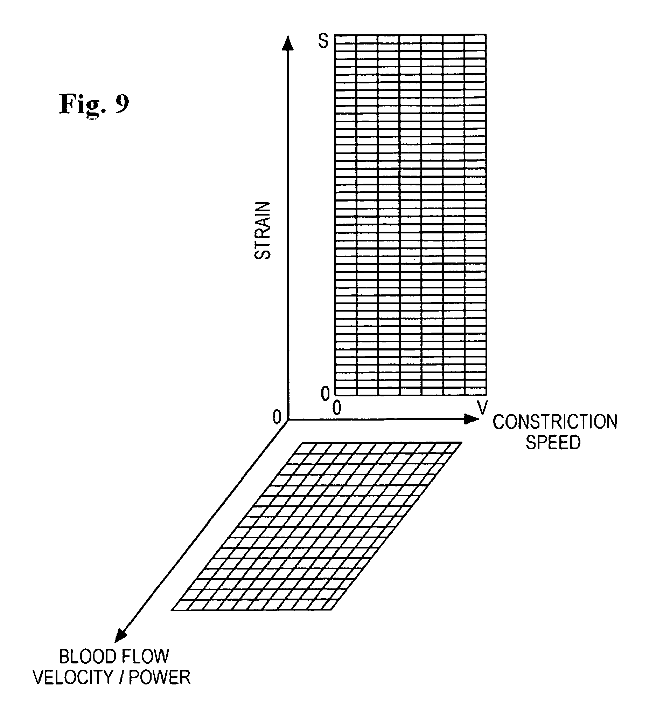

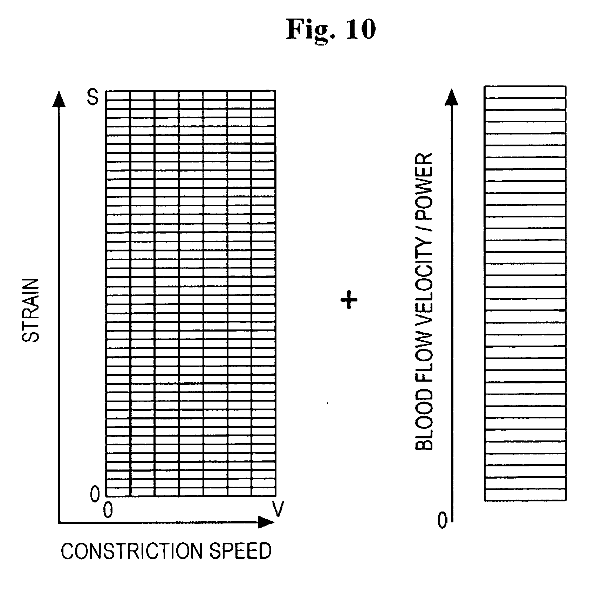

The Doppler image map and the elastic image map can be displayed together with the synthesized image. The Doppler image map and the elastic image map can be represented as a single map. The strain ratio and the constriction speed of the elastic image, as well as the blood flow and the power, are configured 3-dimensionally and shown as a single color map, as shown in Fig. 9. Alternatively, as shown in Fig. 10, the Doppler image map (left side of Fig. 10), which indicates the strain ratio vs. the constriction speed, and the elastic image map (right side of Fig. 10) can be shown separately.

As described above, the present invention can provide specific information on the strain ratio of the elastic image. That is, the information on the constriction speed and the strain ratio can be observed by referring to a single pseudo color map, which provides the images of the constriction speed and the strain ratio simultaneously to allow the optimum elastic image of the tissue to be obtained.

Further, the trial and error due to the mistake of the user can be reduced in the process of obtaining the elastic image to provide an optimum and stable elastic image.

Furthermore, the result of the strain ratio can be displayed with numerical values to show an elasticity difference between the tissues and help diagnose a diseased portion having a minute elasticity difference.

Moreover, the elastic image and the Doppler image are represented simultaneously so that the hard tissue such cancer cell can be observed along with the blood vessel developed around the tissue, which is beneficial to the diagnosis.

When a high intensity focused ultrasound (HIFU) is adopted, the ultrasound diagnostic system constructed in accordance with the present invention may be used to observe the remaining blood vessel after burning the diseased portion by a high intensity ultrasonic wave.

In the ultrasound diagnosis system constructed in accordance with the present invention, the information on the constriction speed can be observed by referring to a single color map to allow the user to obtain the optimized elastic image.

Further, the frame having a high flash noise can be removed to obtain a high quality of the image.

While the present invention has been shown and described with respect to a preferred embodiment, those skilled in the art will recognize that various changes and modifications may be made without departing from the scope of the invention as defined in the appended claims.

## Claims

1. An ultrasound diagnostic system, comprising:
a probe (201) for receiving first echo signals from a target object, the probe being configured to transfer a pressure applied by an user to the target object and further receive second echo signals from the target object;
an elastic image processor (203A) for producing an elastic image and additional information related thereto based on the first and second echo signals;
a Doppler image processor (203B) for producing a Doppler image and a Doppler map based on the echo signals; the system being **characterised by** further comprising:
a synthesis image processor (203C) for producing a synthesized image by synthesizing the elastic image and the Doppler image, the synthesis image processor being configured to receive the additional information and information on the Doppler map; and
a display unit (204) for displaying the synthesized image, the additional information and the Doppler map inputted from the synthesis image processor.

2. The system of Claim 1, wherein the Doppler image processor is configured to check a quantity of a flash noise occurrence in each frame of the Doppler image and removes the frame having the flash noise over a threshold value, the Doppler image processor being configured to replace the removed frame with a new frame formed by an interpolation by using frames before and after the removed frame.

3. The system of Claim 1, wherein the additional information includes an elastic image map and a strain ratio of a medium.

4. The system of Claim 2, further comprising a controller (202) for providing a previously set frame rate to the elastic image processor.

5. The system of Claim 4, wherein the elastic image processor is configured to calculate the strain ratio based on the echo signals and information related to the pressure and is configured to calculate a constriction speed based on the strain ratio and the frame rate, the elastic image processor being configured to form an elastic image map showing a relationship between the constriction speed and the strain ratio.

6. The system of any one of Claims 1 to 5, further comprising a user interface unit (205) for receiving a selection region from the user, wherein the elastic image processor provides information on the strain ratio of the selection region.

## Patentansprüche

1. Diagnostisches Ultraschallsystem, welches Folgendes aufweist:
einen Prüfkopf (201) zum Empfangen erster Echosignale von einem Zielobjekt, wobei der Prüfkopf in der Lage ist, einen von einem Benutzer auf das Zielobjekt aufgebrachten Druck zu übertragen und
des weiteren zweite Echosignale von dem Zielobjekt zu empfangen;
eine Verarbeitungseinrichtung bzw. einen Prozessor (203A) für ein elastisches Bild zum Erzeugen eines elastischen Bilds und zusätzlicher damit verbundener Informationen basierend auf den ersten und zweiten Echosignalen;
einen Dopplerbildprozessor (203B) zum Erzeugen eines Dopplerbilds und eines Dopplerspeicherabbilds basierend auf den Echosignalen;
wobei das System **dadurch gekennzeichnet ist, dass** es des weiteren aufweist:
einen Synthesebildprozessor (203C) zum Erzeugen eines synthetischen Bildes durch Synthetisieren des elastischen Bilds und des Dopplerbilds, wobei der Synthesebildprozessor in der Lage ist, die zusätzlichen Informationen und Informationen bezüglich des Dopplerspeicherabbilds zu empfangen; und
eine Anzeigeeinheit (204) zum Anzeigen des synthetischen Bilds, der zusätzlichen Informationen und
des Dopplerspeicherabbilds, die von dem Synthesebildprozessor eingegeben wurden.

2. System nach Anspruch 1, wobei der Dopplerbildprozessor in der Lage ist, ein Maß des Auftretens von Blitzrauschen in jedem Bildfeld des Dopplerbilds zu prüfen und das Bildfeld zu entfernen, welches das Blitzrauschen über einem Grenzwert aufweist,
wobei der Dopplerbildprozessor in der Lage ist, das entfernte Bildfeld mit einem neuen Bildfeld zu ersetzen, welches durch eine Interpolation unter Verwendung von Bildfeldern vor und nach dem entfernten Bildfeld gebildet wurde.

3. System nach Anspruch 1, wobei die zusätzlichen Informationen eine Speicherabbildung eines elastischen Bilds und einen Belastungsanteil eines Mediums beinhalten.

4. System nach Anspruch 2, welches des weiteren eine Steuereinheit (202) zum Versorgen des elastischen Bildprozessors mit einer voreingestellten Bildfrequenz aufweist.

5. System nach Anspruch 4, wobei der elastische Bildprozessor in der Lage ist, den Belastungsanteil basierend auf den Echosignalen und Informationen bezüglich des Drucks zu berechnen und in der Lage ist, eine Verengungsgeschwindigkeit basierend auf dem Belastungsanteil und der Bildfrequenz zu berechnen, wobei der elastische Bildprozessor in der Lage ist, ein elastisches Speicherabbild zu erzeugen, das eine Beziehung zwischen der Verengungsgeschwindigkeit und dem Belastungsanteil aufweist.

6. System nach einem der Ansprüche 1 bis 5, welches des weiteren eine Benutzerschnittstelleneinheit (205) zum Empfangen eines Auswahlbereichs von dem Benutzer aufweist, wobei der elastische Bildprozessor Informationen hinsichtlich des Belastungsanteils des ausgewählten Bereichs zur Verfügung stellt.

## Revendications

1. Système de diagnostic par ultrasons comprenant :
une sonde (201) pour recevoir des premiers signaux d'écho d'un objet cible, la sonde étant configurée pour transférer une pression appliquée par un utilisateur à l'objet cible et recevoir en outre des seconds signaux d'écho de l'objet cible ;
un processeur d'image élastique (203A) pour produire une image élastique et une information supplémentaire reliée à cette image basée sur les premier et second signaux d'écho ;
un processeur d'image Doppler (203B) pour produire une image Doppler et une carte Doppler basées sur les signaux d'écho ; le système étant **caractérisé en qu'**il comprend en outre :
un processeur d'image de synthèse (203C) pour produire une image de synthèse en synthétisant l'image élastique et l'image Doppler, le processeur d'image de synthèse étant configuré pour recevoir l'information supplémentaire et l'information sur la carte Doppler ; et
une unité d'affichage (204) pour afficher l'image de synthèse, l'information supplémentaire et la carte Doppler, transférées depuis le processeur d'image de synthèse.

2. Système de la revendication 1, dans lequel le processeur d'image Doppler est configuré pour vérifier une valeur d'une apparition d'un bruit de flash dans chaque image de l'image Doppler et pour éliminer l'image comportant un bruit de flash supérieur à une valeur seuil, le processeur d'image Doppler étant configuré pour remplacer l'image éliminée par une nouvelle image formée par une interpolation en utilisant les images précédant et suivant l'image éliminée.

3. Système de la revendication 1 dans lequel l'information supplémentaire comprend une carte de l'image élastique et un rapport de contrainte d'un milieu.

4. Système de la revendication 2, comprenant en outre une unité de commande (202) pour fournir au processeur d'image élastique une vitesse d'image définie à l'avance.

5. Système de la revendication 4, dans lequel le processeur d'image élastique est configuré pour calculer le rapport de contrainte sur la base des signaux d'écho et d'une information concernant la pression et, est configuré pour calculer une vitesse de compression sur la base du rapport de contrainte et de la vitesse d'image, le processeur d'image élastique étant configuré pour former une carte d'image élastique montrant une relation entre la vitesse de compression et le rapport de contrainte.

6. Système de l'une quelconque des revendications 1 à 5, comprenant en outre un module d'interface utilisateur (205) pour recevoir une région sélectionnée d'un utilisateur, dans lequel le processeur d'image élastique fournit de l'information sur le rapport de contrainte de la région sélectionnée.
